# EUROPEAN PATENT SPECIFICATION

(11) **EP 0 365 190 B1**
(45) Date of publication and mention of the grant of the patent: **29.06.1994**
(21) Application number: 89310293.9
(22) Date of filing: 09.10.1989
(51) Int. Cl.: C07D 471/04, C07D 205/08

(54) **Azetidinone intermediates to carbacephalosporins and process**
Azetidinone als Zwischenprodukte für Carbacephalosporine und Verfahren
Azétidinones comme intermédiaires pour carbacéphalosporines et procédé

(30) Priority: 11.10.1988 US 256538
(43) Date of publication of application: 25.04.1990
(73) Proprietor: ELI LILLY AND COMPANY, Indianapolis, Indiana 46285 (US)
(72) Inventor: Gardner, John Paul, Indianapolis Indiana 46256 (US); Jackson, Billy Grinnell, Indianapolis Indiana 46208 (US); Aikins, James, Indianapolis Indiana 46256 (US); Tao, Eddie V., Indianapolis Indiana 46260 (US)
(74) Representative: Tapping, Kenneth George

(56) References cited:
- EP-A- 0 209 352
- TETRAHEDRON LETTERS vol.24, no. 44, 1983, pages 4837, 4838, GB; M. HATAKANA et al.: "A simple synthesis of (+-)-1-carbacephem derivatives"

## Description

This invention relates to β-lactam antibiotics. In particular, it relates to intermediates of 1-carba(1-dethia)-3-cephem-4-carboxylic acids, and to a process for preparing certain intermediates to such compounds.

Hashimoto et al. in U.S. Patent No. 4,335,211 disclose a class of 1-carbacephalosporins having potent oral activity. These compounds are currently being evaluated for the treatment of various conditions such as the common upper- and lower-respiratory tract infections caused by the pathogen H. influenzae. One such compound, known as loracarbef or LY163892, has shown activity against a broad spectrum of bacteria in laboratory tests. Loracarbef has proven to be a relatively stable compound which exhibits high blood levels and a relatively long half-life. Loracarbef is currently being clinically evaluated.

The 1-carbacephalosporins thus far have not been obtained from natural sources, for example, as microbial metabolites. Accordingly, methods for the total synthesis of these promising compounds are highly desirable, particularly methods which are adaptable to large scale manufacture.

The present invention provides a process for preparing a compound of the Formula (I) 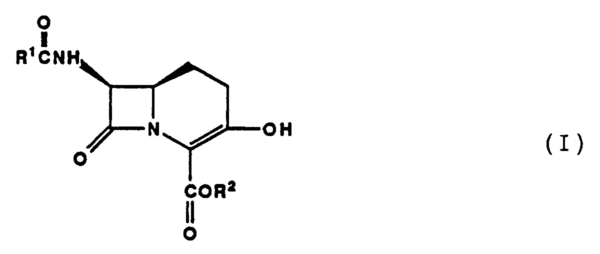
wherein R¹ is C₁-C₆ alkyl; a phenyl group 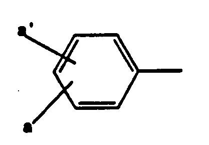
wherein a and a′ independently are hydrogen, C₁-C₄ alkyl, C₁-C₄ alkoxy or halogen; a group represented by the formula 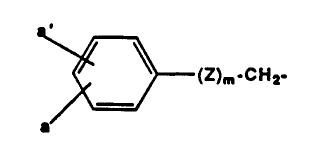
wherein Z is O or S, m is 0 or 1, and a and a′ have the same meanings as defined above; or R¹ is R¹′O wherein R¹' represents C₁-C₄ alkyl, C₅-C₇ cycloalkyl, benzyl, nitrobenzyl, methoxybenzyl, or halobenzyl; and R² is a carboxy-protecting group,
which comprises reacting a compound of the Formula (II) 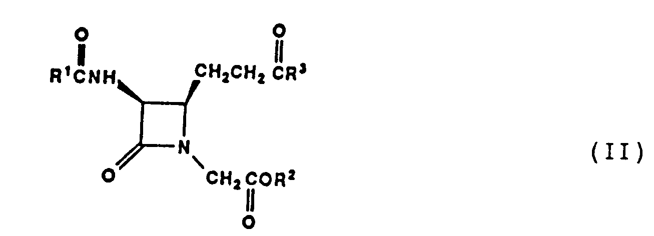
wherein R¹ and R² are as defined above and R³ is a leaving group comprising a substituent which is displaced from the molecule during the chemical reaction and which is based on an oxygen atom, with a non-nucleophilic strong base comprising lithium t-alkoxide, or sodium t-alkoxide.

The present invention also provides intermediates of the Formula (II) 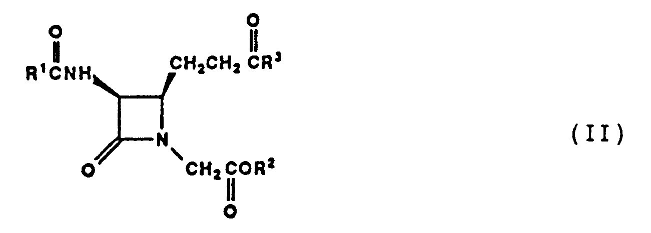
wherein R¹, R² and R³ are as defined above.

A "carboxy-protecting group" as used herein refers to a carboxy function bonded to one of the carboxylic acid substituents commonly employed to block or protect the carboxylic acid functionality while reacting other functional groups on the compound. Examples of such carboxylic acid protecting groups include methyl, t-butyl, phenyl, 4-nitrobenzyl, 4-methoxybenzyl, diphenylmethyl, benzyl, 2,4,6-trimethoxybenzyl, trityl, 4-methoxytrityl, 4,4′-dimethoxytrityl, and 4,4′,4˝-trimethoxytrityl and like moieties. The species of carboxy-protecting group employed is not critical so long as the derivatized carboxylic acid is stable to the condition of subsequent reaction(s) and can be removed at the appropriate point without disrupting the remainder of the molecule. In particular, neither the protected carboxy function nor the protecting group itself should react with the solvents, reagents, products and other substrate molecules of the process aspect of the invention described below. Similar carboxy-protecting groups such as those described by E. Haslam in "Protective Groups in Organic Chemistry" J.F.W. McOmie, Ed., Plenum Press, New York, N.Y., 1973, Chapter 5, and T.W. Greene, "Protective Groups in Organic Synthesis", John Wiley and Sons, New York, N.Y., 1981, Chapter 5 are suitable.

A "leaving group", as defined herein, refers to a substituent which is displaced from the molecule during the chemical reaction and which is based on an oxygen atom. Such a leaving group may be defined as -O-R⁴ where R⁴ represents C₁-C₆ alkyl, C₂-C₆ alkenyl, phenyl, or phenyl substituted with one, two or three substituents such as C₁-C₆ alkyl, C₁-C₆ alkoxy, C₁-C₆ alkylthio, nitro, halo, carboxy, amido and the like, and related substituents. Preferred R⁴ groups include phenyl, halophenyl and nitrophenyl, especially 4-nitrophenyl.

"C₁-C₆ Alkyl" represents a straight or branched alkyl chain having from one to six carbon atoms. Typical C₁-C₆ alkyl groups include methyl, ethyl, n-propyl, isopropyl, n-butyl, sec.-butyl, n-pentyl and n-hexyl.

"C₂-C₆ Alkenyl" represent a straight or branched alkenyl chain having from 2 to 6 carbon atoms. Typical C₂-C₆ alkenyl groups include vinyl, allyl, 2-butenyl, 3-pentenyl, 2-hexenyl and the like.

While the entire scope of process variables taught herein are believed operable, the present process and intermediates do have preferred aspects. Preferred compounds of the above formula are where R¹ is benzyl, phenyl, benzyloxy or methylenephenoxy (wherein m is 1, Z is oxygen and a and a′ are both hydrogen) and R² is alkyl, especially methyl, or substituted benzyl, especially 4-nitrobenzyl and 4-methoxybenzyl. Other preferred aspects of the present invention will be noted hereinafter.

The process of the present invention is carried out by combining at least one, but preferably at least three, molar equivalents of base with the compound of Formula (II) in a suitable organic solvent. More specifically, the process is carried out by first preparing a solution of the base and a suitable solvent. A variety of organic solvents may be used with the aprotic solvents being preferred. Typical aprotic solvents suitable for use in the reaction include the ethers such as 1,2-dimethoxyethane and diethyl ether, and the cyclic ethers, such as tetrahydrofuran (THF), which is preferred. In general, the solvent employed in the present process can be any convenient solvent which solubilizes the azetidinone starting material and which is unreactive with the base employed in the reaction. The amount of solvent used in preparing the solution is not critical, but no more than necessary to dissolve the base need be used.

Non-nucleophilic strong bases are employed in the process of the present invention. Typical non-nucleophilic strong bases include the alkali metal alkoxides of tertiary alcohols such as lithium t-butoxide, potassium t-butoxide, sodium t-butoxide, sodium t-pentoxide, and potassium t-pentoxide, metal hydrides such as potassium hydride and sodium hydride, and other non-nucleophilic strong bases such as lithium hexamethyldisilazide and lithium diisopropylamide.

The process of the present invention is preferably carried out under substantially anhydrous conditions. The term "substantially anhydrous conditions", as used herein, represents reaction conditions which are virtually free from water due to the reactivity of the base with water. Accordingly, solvents are preferably dried prior to use in the present invention and the base is kept moisture free prior to its use. Further, it is preferred that all reaction vessels be thoroughly dried prior to their use, for example by flame or heat drying. It is also preferred that the process be carried out in an inert atmosphere, such as under nitrogen or argon gas.

The solution of base thus prepared is next cooled to a temperature in the range of about 25°C to about -100°C, more preferably from about -50°C to about -80°C.

Next, the compound of Formula (II) is combined with the base. Preferably, a solution of the azetidinone of Formula (II) is added to the cold solution of base. Generally, the same solvent used to solubilize the base is used to solubilize the azetidinone, but such is not required.

The reaction is substantially complete within about 10 minutes to 24 hours when conducted at a temperature in the range of about -100°C to about 25°C. The desired compound is readily isolated by quenching the mixture with acid, such as hydrochloric acid or acetic acid, preferably at a temperature in the range of about -80°C to about -60°C. Typically, the mixture is then warmed to about 25°C and a small amount of water added. The desired compound may be isolated by extraction with a water immiscible organic solvent or crystallization by the addition of common solvents and collecting the solid. The isolated residue may be further purified if desired by standard techniques such as crystallization from common solvents or column chromatography over solid supports such as silica gel or alumina.

As noted above the 7β-amino-3-hydroxy-1-carba(1-dethia)-3-cephem-4-carboxylic acid esters prepared by the process of the present invention are valuable intermediates to 1-carbacephalosporin antibiotics, more particularly to 3-halo-1-carba-3-cephem compounds disclosed in U.S. Patent No. 4,708,956, incorporated herein by reference. The 3-hydroxy-1-carbacephalosporins are converted to the 3-halo antibiotics according to the process disclosed in U.S. Patent No. 4,673,737, also herein incorporated by reference. According to this process a 7-(protected amino)-3-hydroxy-1-carbacephalosporin ester is reacted with an acylating reagent which is a derivative of trifluoromethane sulfonic acid, such as trifluoromethanesulfonic anhydride or trifluoromethanesulfonyl chloride, and the like. The resulting 3-trifluoromethylsulfonyloxy-1-carba-3-cephems are reacted with a lithium halide to provide the corresponding 3-halo-1-carbacephalosporin antibiotic.

The cis-3-(substituted amino)-1-(2-substituted 2-oxoethyl)-4-azetidin-2-one propanoic acid esters employed as starting materials in the process of the present invention may be prepared by acylating the amino group of a 3β-(substituted amino)-4-[2-(2-furyl)ethyl]azetidin-2-one followed by N-substitution of the azetidinone with a substituted haloacetate. The resulting compound is then ozonolyzed to the 2-carboxyethyl N-alkylated protected aminoazetidinone, which is finally converted to the desired cis-3-(substituted amino)-1-(2-substituted 2-oxoethyl)-4-azetidin-2-one propanoic acid ester. These reactions may be represented by the following scheme: 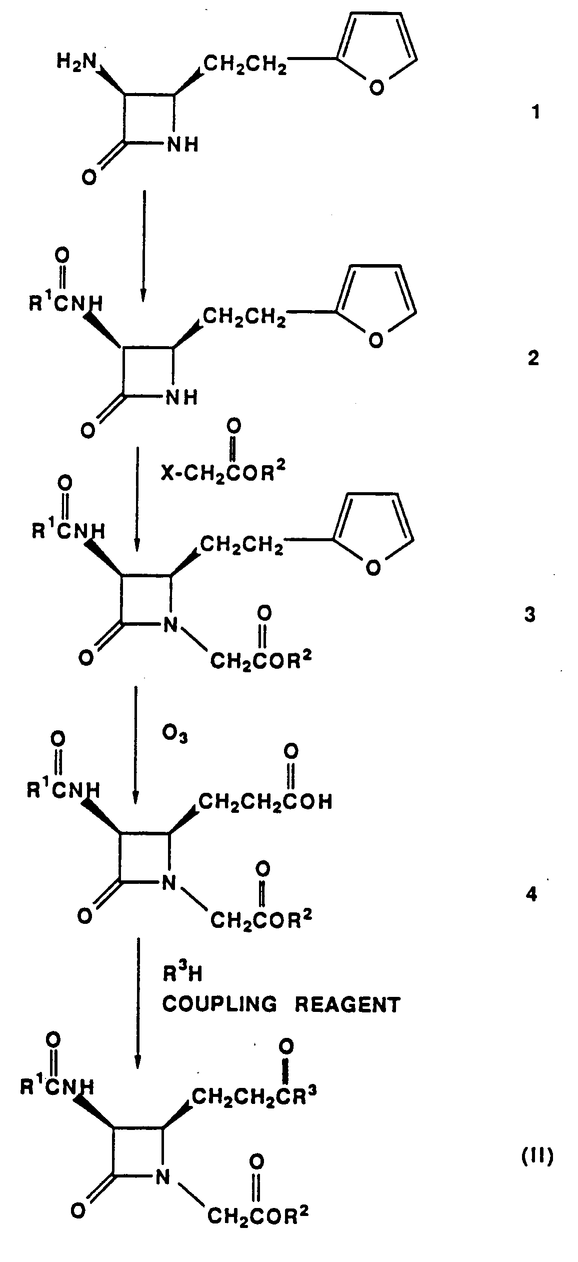
The 3β-amino-4-[2-(2-furyl)ethyl]azetidin-2-one (1) is a known compound readily prepared by prior art processes. For example, Evans et al. in U.S. Patent No. 4,665,171, incorporated herein by reference, teaches the synthesis of this compound by reducing a 4β-[2-(2-furyl) ethyl]-3β-(4(S)-aryloxazolidin-2-one-3-yl)azetidinone with lithium in ammonia in the presence of about three molar equivalents of t-butyl alcohol.

The 3β-(substituted amino)-4-[2-(2-furyl)ethyl]azetidin-2-one (2) may be prepared by acylating the 3β-amino substitutent of the compound of formula (1) under standard acylating conditions.

The 1-(2-substituted 2-oxoethyl)-3β-(substituted amino)-4-[2-(2-furyl)ethyl]azetidin-2-one (3) is prepared by reacting the compound of formula (2) with a substituted haloacetate in the presence of an acid scavenger. Typical substituted haloacetates include substituted bromo- or chloroacetates, whereas commonly used acid scavengers include bases such as sodium hydroxide and related bases.

Ozonolysis of (3) provides the 2-carboxyethyl N-alkylated protected aminoazetidinone (4), which is finally converted to the desired compound of Formula (II) cis-3-(substituted amino)-1-[(2-substituted 2-oxoethyl]-4-azetidin-2-one propanoic acid ester employing an alcohol R³H, wherein R³ is as defined above, and a coupling reagent under standard conditions. Examples of such coupling reagents include the carbodiimides such as N,N′-dicyclohexylcarbodiimide, N,N′-diisopropylcarbodiimide, or N,N′-diethylcarbodiimide; the imidazoles such as carbonyldiimidazole; as well as reagents such as N-ethoxycarbonyl-2-ethoxy-1,2-dihydroquinoline (EEDQ).

The following Examples further illustrate specific aspects of the present invention. The Examples are not intended to be limiting in any respect and should not be so construed. In the following Examples the term "IR" stands for "Infrared Spectrum", "UV" represents "Ultraviolet Spectrum", and "FDMS" represents "Field Desorption Mass Spectrum".

### Example 1

### Synthesis of 7-[[(phenylmethoxy)carbonyl]amino]-3-hydroxy-8-oxo-1-azabicyclo[4.2.0]oct-2-ene-2-carboxylic acid (4-methoxyphenyl)methyl ester

A 50 ml three neck round bottom flask fitted with a nitrogen inlet tube, thermometer, nitrogen outlet tube, addition funnel and magnetic stirrer was charged with 0.39 g (3.5 mmol) of potassium t-butoxide and 15 ml of THF under a nitrogen atmosphere. The resulting solution was cooled to about -78°C with an external dry ice/acetone bath and a solution of 0.55 g (1.0 mmol) of cis-3-[[(phenylmethoxy)carbonyl]amino]-1-[2-[(4-methoxyphenyl)methoxy]-2-oxoethyl]-4-azetidin-2-one propanoic acid phenyl ester in 5 ml of THF was added dropwise over a period of four minutes. The yellow solution was stirred at about -75°C for about 30 minutes and 0.29 ml (5.0 mmol) of glacial acetic acid was added. The mixture was allowed to warm to room temperature over a period of ten minutes and 30 ml of water was added. The resulting mixture was extracted with 50 ml of ethyl acetate. The organic layer was separated, dried over anhydrous sodium sulfate and concentrated under vacuum to provide 2.38 g of a solution. To the solution was added 15 ml of diethyl ether followed by 15 ml of hexanes. The mixture was stirred for one hour, and the precipitated solid was collected by vacuum filtration and washed with 10 ml of hexanes:diethyl ether (1:1, v:v). The solid was dried in a vacuum oven at 40°C to provide 0.32 g of 7-[[(phenylmethoxy)carbonyl]amino]-3-hydroxy-8-oxo-1-azabicyclo[4.2.0]oct-2-ene-2-carboxylic acid (4-methoxyphenyl)methyl ester as a white solid.

| Analysis calculated for C₂₄H₂₄N₂O₇ | | | |
|---|---|---|---|
| Theory: | C, 63.71; | H, 5.35; | N, 6.19; |
| Found: | C, 63.98; | H, 5.51; | N, 6.13. |

IR (CHCl₃): 3439, 1766, 1725, 1663, 1614, 1516, 1387, 1338, 1050 cm⁻¹;
UV (CH₃CH₂OH): λ max 280 nm (7560), 226 nm (14,600 );
FDMS: 452 = M+

### Example 2

### Synthesis of 7-(benzoylamino)-3-hydroxy-8-oxo-1-azabicyclo[4.2.0]oct-2-ene-2-carboxylic acid (4-methoxyphenyl)methyl ester

A. cis-3-(Benzoylamino)-1-[2-[(4-methoxyphenyl)methoxy]-2-oxoethyl]-4-azetidin-2-one propanoic acid (4-nitrophenyl) ester
A mixture of 1.5 g (3.42 mmol) of cis-3-(benzoylamino)-1-[2-[(4-methoxyphenyl)methoxy]-2-oxoethyl]-4-azetidin-2-one propanoic acid, 0.81 g (5.1 mmol) of carbonyldiimidazole and 30 ml of dry THF was stirred at room temperature for one hour. To the mixture was added 0.71 g (5.1 mmol) of p-nitrophenol and the resulting mixture was stirred at room temperature overnight. The mixture was concentrated under vacuum and the residue was dissolved in 50 ml of ethyl acetate. The resulting solution was washed with 30 ml of 1N hydrochloric acid, 20 ml of a saturated sodium chloride solution and 50 ml of water. The organic phase was dried over anhydrous magnesium sulfate and concentrated under vacuum to provide 1.72 g of a yellow oil. The oil was chromotographed over silica gel employing hexane/ethyl acetate as the eluant to provide 0.4 g of a white solid following evaporation under vacuum. mp = 146°-152°C.

| Analysis calculated for C₂₉H₂₇N₃O₉ | | | |
|---|---|---|---|
| Theory: | C,62.03; | H,4.85; | N,7.48; |
| Found: | C,62.27; | H,4.92; | N,7.20. |

B. A solution of 10 ml of THF and 0.17 g (1.54 mmol) of potassium t-butoxide under a nitrogen atmosphere in a 100 ml three neck round bottom flask was cooled to about -78°C with an external dry ice/acetone bath. To this solution was added a solution of 0.27 g (0.48 mmol) of cis-3-(benzoylamino)-1-[2-[(4-methoxyphenyl)methoxy]-2-oxoethyl]-4-azetidin-2-one propanoic acid (4-nitrophenyl) ester in 3 ml of dry THF dropwise over a period of about 5 minutes. The resulting yellow solution was stirred for about 50 minutes at about -78°C and 30 ml of 1N hydrochloric acid, 20 ml of a saturated sodium chloride solution and 50 ml of ethyl acetate were added. The organic layer was separated, washed with water, dried over anhydrous magnesium sulfate and concentrated under vacuum to provide 0.28 g of 7-(benzoylamino)-3-hydroxy-8-oxo-1-azabicyclo[4.2.0]oct-2-ene-2-carboxylic acid (4-methoxyphenyl)methyl ester as an oil.
H-NMR(DMSO): δ2.10 (multiplet, 2H); 2.70 (multiplet, 2H); 3.96 (singlet, 3H); 4.07 (multiplet, 1H); 5.49 (singlet, 2H); 5.76 (quartet, 1H); 7.18 and 7.70 (multiplet, 4H); 7.82 and 8.20 (multiplet, 5H); 9.40 (doublet, 1H).

### Example 3

### Synthesis of 7-(benzoylamino)-3-hydroxy-8-oxo-1-azabicyclo[4.2.0]oct-2-ene-2-carboxylic acid (4-methoxyphenyl)methyl ester

A. cis-3-(Benzoylamino)-1-[2-[(methoxyphenyl) methoxy]-2-oxoethyl]-4-azetidin-2-one propanoic acid (2,4,5-trichlorophenyl)methyl ester
A mixture of 1.5 g (3.42 mmol) of cis-3-(benzoylamino)-1-[2-[(4-methoxyphenyl)methoxy]-2-oxoethyl]-4-azetidin-2-one propanoic acid, 0.81 g (5.1 mmol) of carbonyldiimidazole and 30 ml of dry THF was stirred at room temperature for two hours. To this mixture was added 1.0 g (5.1 mmol) of 2,4,5-trichlorophenol and the resulting mixture was stirred at room temperature for 19 hours. The mixture was heated to reflux for 30 minutes and stirred for an additional 4½ hours at room temperature. The reaction mixture was concentrated under vacuum, and the residue was dissolved in ethyl acetate. The resulting solution was washed with 1N hydrochlric acid, dried over anhydrous magnesium sulfate and concentrated under vacuum to provide 2.34 g of an oil. The oil was chromatographed over silica gel while eluting with hexane/ethyl acetate to provide 0.46 g of cis-3-(benzoylamino)-1-[2-[(methoxyphenyl)methoxy]-2-oxoethyl]-4-azetidin-2-one propanoic acid (2,4,5-trichlorophenyl)methyl ester.

| Analysis calculated for C₂₉H₂₅Cl₃N₂0₇ | | | | |
|---|---|---|---|---|
| Theory: | C,56.19; | H,4.07; | N,4.52; | Cl, 17.16; |
| Found: | C,56.12; | H,4.14; | N,4.35; | Cl,16.91. |

B. A 100 ml three neck round bottom flask was charged with 0.27 g (2.37 mmol) of potassium t-butoxide and 15 ml of THF under a nitrogen atmosphere. The resulting solution was cooled to about -78°C with an external dry ice/acetone bath and a solution of 0.46 g (0.74 mmol) of cis-3-(benzoylamino)-1-[2-[(4-methoxyphenyl)methoxy]-2-oxoethyl]-4-azetidin-2-one propanoic acid (2,4,5-trichlorophenyl)methyl ester in 5 ml of THF was added dropwise. The reaction mixture was stirred for about 210 minutes at about -78°C and quenched with 30 ml of 1N hydrochloric acid, 20 ml of an aqueous saturated sodium chloride solution and 50 ml of ethyl acetate. The organic layer was separated, washed with water, dried over anhydrous magnesium sulfate and concentrated under vacuum to provide 0.37 g of an oil containing 7-(benzoylamino)-3-hydroxy-8-oxo-1-azabicyclo[4.2.0]oct-2-ene-2-carboxylic acid (4-methoxyphenyl)methyl ester. The structure of the compound was confirmed by thin layer chromatography.

### Example 4

### Synthesis of 7-(benzoylamino)-3-hydroxy-8-oxo-1-azabicyclo[4.2.0]oct-2-ene-2-carboxylic acid (4-methoxyphenyl)methyl ester

Fifteen milliliters of THF were added to 0.35 g (3.1 mmol) of potassium t-butoxide under a nitrogen atmosphere in a three neck round bottom flask. The resulting solution was cooled to about -78°C with an external dry ice/acetone bath and charged with a solution of 0.5 g (0.97 mmol) of cis-3-(benzoylamino)-1-[2-[(4-methoxyphenyl)methoxy]-2-oxoethyl]-4-azetidin-2-one propanoic acid (4-nitrophenyl) ester in 5 ml of THF via syringe. The resulting yellow solution was stirred for about 30 minutes and 0.29 ml (5.0 mmol) of glacial acetic acid was added. The mixture was allowed to warm to room temperature for five minutes and 30 ml of water and 50 ml of ethyl acetate was added. The organic layer was separated, dried over anhydrous magnesium sulfate and concentrated under vacuum to provide 2.2 g of 7-(benzoylamino)-3-hydroxy-8-oxo-1-azabicyclo[4.2.0]-oct-2-ene-2-carboxylic acid (4-methoxyphenyl)methyl ester as an oil. Five milliliters of diethyl ether were added to the oil and the solution was seeded with a crystal of the desired compound. An additional 10 ml of diethyl ether was added and the mixture was stirred for 10 minutes. Next, 10 ml of hexanes was added dropwise over a period of five minutes and the mixture was stirred for one hour at room temperature. The precipitated solid was collected by vacuum filtration, washed with diethyl ether:hexane (3:2, v:v) and dried under vacuum to give 0.30 g of the title compound as off-white crystals. The structure of the compound was confirmed by thin layer chromatography.

### Example 5

### Synthesis of 7-(benzoylamino)-3-hydroxy-8-oxo-1-azabicyclo[4.2.0]oct-2-ene-2-carboxylic acid (4-methoxyphenyl)methyl ester

A 300 ml two neck round bottom flask was charged with 3.35 g (31.2 mmol) of potassium t-butoxide under nitrogen. Tetrahydrofuran (150 ml) was added to the flask and the resulting solution was cooled to about -78°C with an external dry ice/acetone bath. In a separate container 5.0 g (8.9 mmol) of cis-3-(benzoylamino)-1-[2-[(4-methoxyphenyl)methoxy-2-oxoethyl]-4-azetidin-2-one propanoic acid (4-nitrophenyl) ester was dissolved in 35 ml of THF under nitrogen. The resulting solution was added via syringe to the flask containing the potassium t-butoxide solution over a period of about 10 minutes. The reaction mixture was stirred at -78°C for about one hour and a thin layer chromatograph employing toluene:ethyl acetate (7:3, v:v) with 1 ml of acetic acid indicated that no starting material remained. The reaction mixture was allowed to warm to about 0°C and 2.5 ml of glacial acetic acid was added. The mixture was stirred for about 30 minutes and concentrated under vacuum at about 40°C. The resulting oil was dissolved in methylene chloride and the resulting solution was washed five times with 10% by volume aqueous saturated sodium bicarbonate solution, once with water and dried over anhydrous magnesium sulfate. The solution was concentrated under vacuum to dryness to provide 3.47 g of the desired compound as compared by thin layer chromatography to an authentic reference standard.

### Example 6

### Synthesis of 7-[[(phenylmethoxy)carbonyl]amino]-3-hydroxy-8-oxo-1-azabicyclo[4.2.0]oct-2-ene-2-carboxylic acid (4-nitrophenyl)methyl ester

A solution of 0.56 g (4.99 mmol) of potassium t-butoxide in 10 ml of dry THF was cooled to about -74°C with an external dry ice/acetone bath under a nitrogen atmosphere. To the reaction mixture was added a solution of 0.8 g (1.43 mmol) in 10 ml of dry THF dropwise over a period of about 10 minutes. The reaction mixture was stirred at about -70°C for about 10 minutes and 0.409 ml (7.2 mmol) of glacial acetic acid was added. The reaction mixture was allowed to warm to room temperature with stirring and 50 ml of ethyl acetate, 25 ml of a saturated aqueous sodium chloride solution and 25 ml of a saturated aqueous sodium bicarbonate solution was added. The organic layer was separated, dried over anhydrous magnesium sulfate and partially concentrated under vacuum. The residue was stirred at room temperature and a solid began to precipitate out of solution. The solid was collected by vacuum filtration to provide 180 mg of the title compound.

| Analysis calculated for C₂₃H₂₉N₃0₈ | | | |
|---|---|---|---|
| Theory: | C, 59.23; | H, 4.32; | N, 9.01; |
| Found: | C, 59.45, | H, 4.55; | N, 9.02. |

### Example 7

### Synthesis of 7-[[(phenylmethoxy)carbonyl]amino]-3-hydroxy-8-oxo-1-azabicyclo[4.2.0]oct-2-ene-2-carboxylic acid (4-nitrophenyl)methyl ester

A. Using sodium t-butoxide as base
A 0.67 g (9 mmol) sample of t-butanol was dissolved in 8 ml of dry tetrahydrofuran at -10°C. The solution was then treated with 0.36 g (9 mmol) of NaH and allowed to warm to room temperature and finally heated to reflux. The reaction mixture was then cooled to -74°C and treated with a 1.00 g (m.78 mmol) sample of cis-3(benzoylamino)-1-[2-[(4-methoxyphenyl)methoxy-2-oxoethyl]-4-azetidin-2-one propanoic acid (4-nitrophenyl) ester. The reaction was then quenched after a short time with 100 ml of 1N HCl and extracted with 100 ml of CH₂Cl₂. The organic phase was then dried over anhydrous Na₂SO₄, filtered, concentrated in vacuo, and crystallized from methyl-t-butyl ether to provide 0.63 g of the title compound as an off-white solid.
(yield = 75.7%)
mp = 149-152°C

| Elemental Analysis: | | |
|---|---|---|
| | Theoretical (%) | Found (%) |
| C: | 59.1 | 59.07 |
| H: | 4.53 | 4.58 |
| N: | 8.99 | 8.97 |

NMR analysis corresponded to that of authentic material made according to the foregoing examples.
B. Using sodium t-pentoxide as base
A 14.72 g (133.69 mmol) sample of sodium t-pentoxide was dissolved in 70 ml of dry tetrahydrofuran and cooled to -70°C. The solution was then treated with a 15.0g (20.74 mmol) sample of cis-3(benzoylamino)-1-[2-[(4-methoxyphenyl)methoxy-2-oxoethyl]-4-azetidin-2-one propanoic acid (4-nitrophenyl) ester and the reaction maintained below -50°C for 35 minutes. The reaction mixture was quenched with 150 mmol of 1N HCl and with 8.0 ml H₂O. The mixture was then warmed to -20°C and treated with 70 ml of saturated NaCl solution. The reaction mixture was then extracted with 150 ml of CH₂Cl₂. The organic phase was then dried over anhydrous MgSO₄, filtered and concentrated in vacuo.
Crystallization from t-butyl methylether afforded 11.01 g (88% yield) of the title compound.
mp = 160-162°C

| Elemental Analysis: | | |
|---|---|---|
| | Theoretical (%) | Found (%) |
| C: | 59.1 | 59.05 |
| H: | 4.53 | 4.79 |
| N: | 8.99 | 9.15 |

Mass Spectrum (FD) m/e = 467

## Claims (Claims for the following Contracting State(s): AT, BE, CH, DE, FR, GB, GR, IT, LI, NL, SE)

1. A process for preparing a compound of the formula 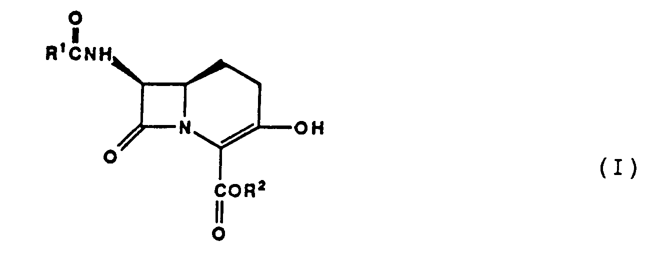 wherein R¹ is C₁-C₆ alkyl; a phenyl group 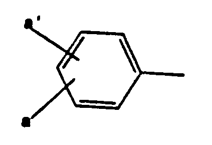 wherein a and a' independently are hydrogen, C₁-C₄ alkyl, C₁-C₄ alkoxy or halogen; a group represented by the formula 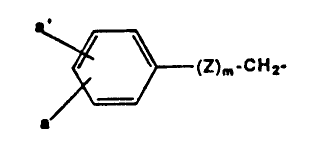 wherein Z is O or S, m is 0 or 1, and a and a' have the same meanings as defined above; or R' is R¹'O wherein R¹' represents C₁-C₄ alkyl, C₅-C₇ cycloalkyl, benzyl, nitrobenzyl, methoxybenzyl, or halobenzyl; and R² is a carboxy-protecting group,
which comprises reacting a compound of the formula 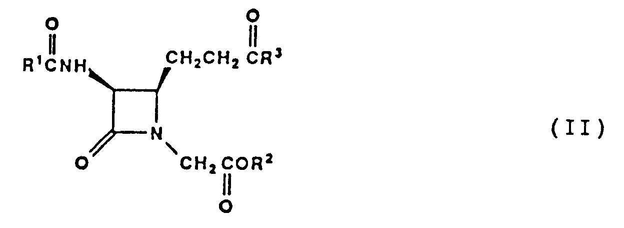 wherein R¹ and R² are as defined above and R³ is a leaving group comprising a substituent which is displaced from the molecule during the chemical reaction and which is based on an oxygen atom, with a non-nucleophilic strong base comprising lithium t-alkoxide, or sodium t-alkoxide.

2. A process according to Claim 1 wherein R¹ is benzyloxy.

3. A process according to Claim 1 or 2 wherein R² is methyl.

4. A process according to Claim 1, 2, or 3 wherein R² is 4-nitrobenzyl.

5. A process according to any one of Claims 1 to 4, wherein R³ is phenoxy.

6. An intermediate of the formula 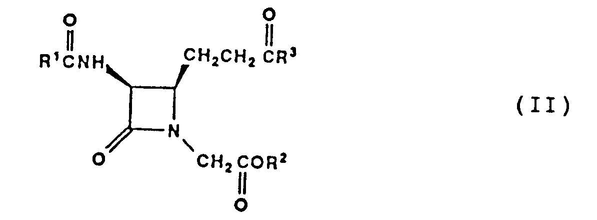 wherein R¹ is C₁-C₆ alkyl; a phenyl group 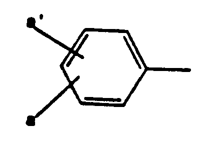 wherein a and a' independently are hydrogen, C₁-C₄ alkyl, C₁-C₄ alkoxy or halogen; a group represented by the formula 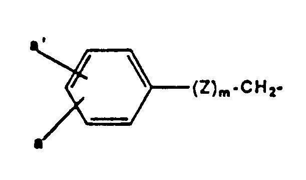 wherein Z is O or S, m is 0 or 1, and a and a' have the same meanings as defined above; or R¹ is R¹'O wherein R¹' represents C₁-C₄ alkyl, C₅-C₇ cycloalkyl, benzyl, nitrobenzyl, methoxybenzyl, or halobenzyl;
R² is a carboxy protecting group; and
R³ is a leaving group.

7. An intermediate as claimed in Claim 6 wherein R¹ is benzyloxy.

8. An intermediate as claimed in Claim 6 wherein R² is 4-nitrobenzyl, methyl, or 4-methoxybenzyl.

9. An intermediate as claimed in any one of Claims 6 to 8 wherein R³ is phenoxy, halophenoxy, or nitrophenoxy.

## Claims (Claims for the following Contracting State(s): ES)

1. A process for preparing a compound of the formula 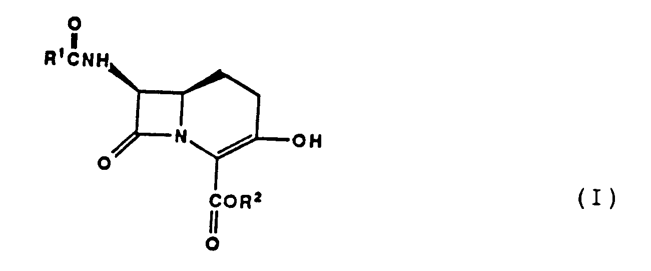 wherein R¹ is C₁-C₆ alkyl; a phenyl group 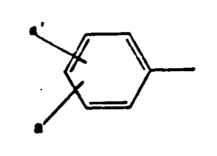 wherein a and a' independently are hydrogen, C₁-C₄ alkyl, C₁-C₄ alkoxy or halogen; a group represented by the formula 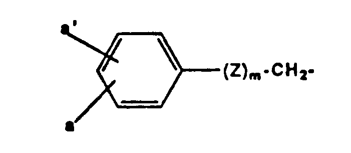 wherein Z is O or S, m is 0 or 1, and a and a' have the same meanings as defined above; or R¹ is R¹'O wherein R¹' represents C₁-C₄ alkyl, C₅-C₇ cycloalkyl, benzyl, nitrobenzyl, methoxybenzyl, or halobenzyl; and R² is a carboxy-protecting group,
which comprises reacting a compound of the formula 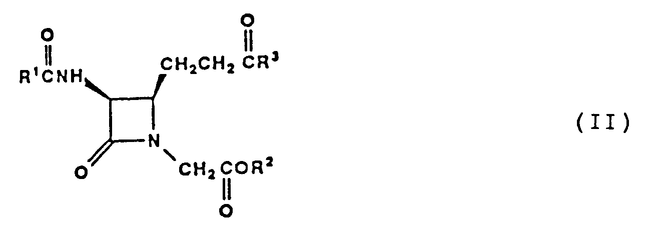 wherein R¹ and R² are as defined above and R³ is a leaving group comprising a substituent which is displaced from the molecule during the chemical reaction and which is based on an oxygen atom, with a non-nucleophilic strong base comprising lithium t-alkoxide, or sodium t-alkoxide.

2. A process according to Claim 1 wherein R¹ is benzyloxy, phenyl, or benzyl.

3. A process according to Claim 1 or 2 wherein R² is methyl.

4. A process according to Claim 1, 2, or 3 wherein R² is 4-nitrobenzyl.

5. A process according to any one of Claims 1 to 4, wherein R³ is phenoxy, halophenoxy, or nitrophenoxy.

## Patentansprüche (Patentansprüche für folgende(n) Vertragsstaat(en): AT, BE, CH, DE, FR, GB, GR, IT, LI, NL, SE)

1. Verfahren zur Herstellung einer Verbindung der Formel 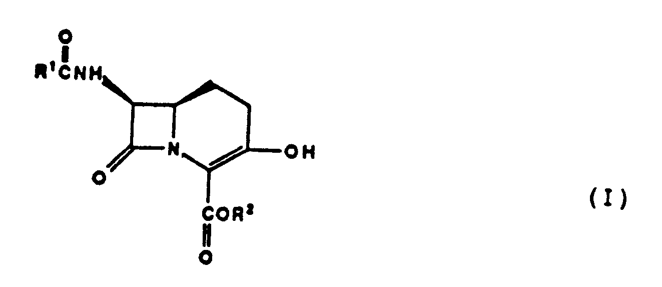 worin R¹ ein C₁-C₆-Alkylrest; eine Phenylgruppe 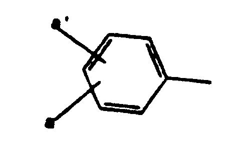 worin a und a' unabhängig Wasserstoff, C₁-C₄-Alkylreste, C₁-C₄-Alkoxyreste oder Halogen sind; eine Gruppe der Formel 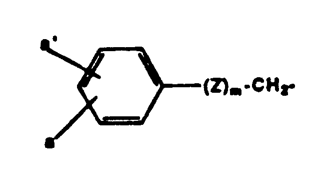 worin Z O oder S ist, m 0 oder 1 ist und a und a' die gleichen Bedeutungen wie oben definiert haben, ist; oder R¹ R¹'O ist, worin R¹' ein C₁-C₄-Alkyl-, C₅-C₇-Cycloalkyl-, Benzyl-, Nitrobenzyl-, Methoxybenzyl- oder Halogenbenzylrest ist; und R² eine Carboxyschutzgruppe ist,
umfassend, daß man eine Verbindung der Formel (II) 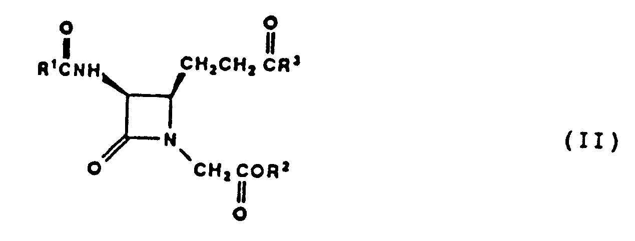 worin R¹ und R² wie oben definiert sind und R³ eine Abgangsgruppe ist, die einen Substituenten umfaßt, der aus dem Molekül während der chemischen Reaktion verdrängt wird und auf einem Sauerstoffatom basiert, mit einer nicht-nukleophilen starken Base, umfassend Lithium-t-alkoxid oder Natrium-t-alkoxid, umsetzt.

2. Verfahren nach Anspruch 1, worin R¹ ein Benzyloxyrest ist.

3. Verfahren nach Anspruch 1 oder 2, worin R² ein Methylrest ist.

4. Verfahren nach einem der Ansprüche 1, 2 oder 3, worin R² ein 4-Nitrobenzylrest ist.

5. Verfahren nach einem der Ansprüche 1 bis 4, worin R³ ein Phenoxyrest ist.

6. Zwischenprodukt der Formel 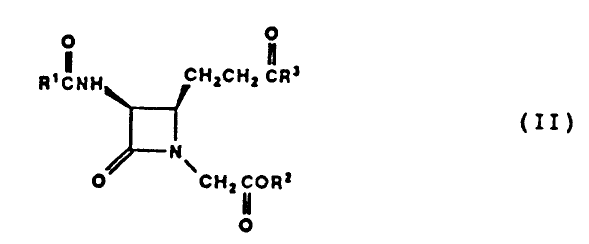 worin R¹ ein C₁-C₆-Alkylrest; eine Phenylgruppe 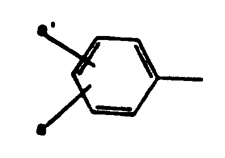 worin a und a' unabhängig Wasserstoff, C₁-C₄-Alkylreste, C₁-C₄-Alkoxyreste oder Halogen sind; eine Gruppe der Formel 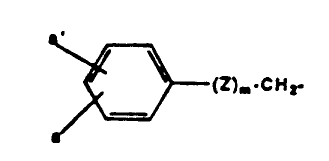 worin Z O oder S ist, m 0 oder 1 ist und a und a' die gleichen Bedeutungen wie oben definiert haben, ist; oder R¹ R¹'O ist, worin R¹' einen C₁-C₄-Alkyl-, C₅-C₇-Cycloalkyl-, Benzyl-, Nitrobenzyl-, Methoxybenzyl- oder Halogenbenzylrest bedeutet; R² eine Carboxyschutzgruppe ist und R³ eine Abgangsgruppe ist.

7. Zwischenprodukt nach Anspruch 6, worin R¹ ein Benzyloxyrest ist.

8. Zwischenprodukt nach Anspruch 6, worin R² ein 4-Nitrobenzyl-, Methyl- oder 4-Methoxybenzylrest ist.

9. Zwischenprodukt nach einem der Ansprüche 6 bis 8, worin R³ ein Phenoxy-, Halogenphenoxy- oder Nitrophenoxyrest ist.

## Patentansprüche (Patentansprüche für folgende(n) Vertragsstaat(en): ES)

1. Verfahren zur Herstellung einer Verbindung der Formel 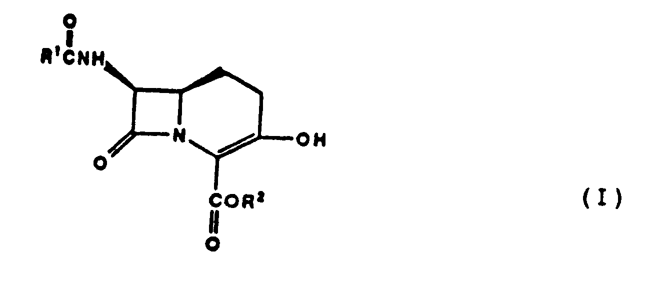 worin R¹ ein C₁-C₆-Alkylrest; eine Phenylgruppe 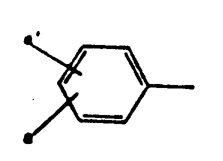 worin a und a' unabhängig Wasserstoff, C₁-C₄-Alkylreste, C₁-C₄-Alkoxyreste oder Halogen sind; eine Gruppe der Formel 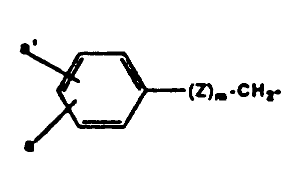 worin Z O oder S ist, m 0 oder 1 ist und a und a' die gleichen Bedeutungen wie oben definiert haben, ist; oder R¹ R¹'O ist, worin R¹' ein C₁-C₄-Alkyl-, C₅-C₇-Cycloalkyl-, Benzyl-, Nitrobenzyl-, Methoxybenzyl- oder Halogenbenzylrest ist; und R² eine Carboxyschutzgruppe ist,
umfassend, daß man eine Verbindung der Formel (II) 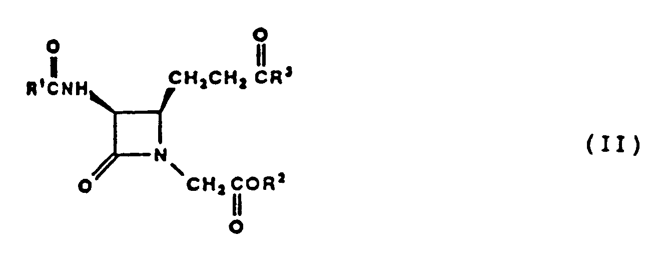 worin R¹ und R² wie oben definiert sind und R³ eine Abgangsgruppe ist, die einen Substituenten umfaßt, der aus dem Molekül während der chemischen Reaktion verdrängt wird und auf einem Sauerstoffatom basiert, mit einer nicht-nukleophilen starken Base, umfassend Lithium-t-alkoxid oder Natrium-t-alkoxid, umsetzt.

2. Verfahren nach Anspruch 1, worin R¹ ein Benzyloxy-, Phenyl- oder Benzylrest ist.

3. Verfahren nach Anspruch 1 oder 2, worin R² ein Methylrest ist.

4. Verfahren nach einem der Ansprüche 1, 2 oder 3, worin R² ein 4-Nitrobenzylrest ist.

5. Verfahren nach einem der Ansprüche 1 bis 4, worin R³ ein Phenoxy-, Halogenphenoxy- oder Nitrophenoxyrest ist.

## Revendications (Revendications pour l'(les) Etat(s) contractant(s) suivant(s): AT, BE, CH, DE, FR, GB, GR, IT, LI, NL, SE)

1. Procédé pour préparer un composé de formule 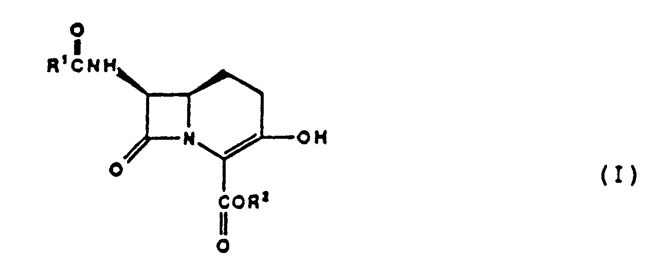 dans laquelle R¹ représente un groupe alkyle en C₁-C₆; un groupe phényle, 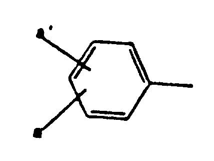 dans lequel a et a' représentent, indépendamment l'un de l'autre, un atome d'hydrogène, un groupe alkyle en C₁-C₄, un groupe alcoxy en C₁-C₄ ou encore un atome d'halogène; un groupe représenté par la formule 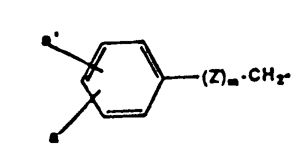 dans laquelle Z représente O ou S, m = 0 ou 1 et a et a' ont les mêmes significations que celles définies ci-dessus; ou bien R¹ représente R¹'O où R¹' représente un groupe alkyle en C₁-C₄, un groupe cycloalkyle en C₅-C₇, un groupe benzyle, un groupe nitrobenzyle, un groupe méthoxybenzyle ou un groupe halogénobenzyle; et R² représente un groupe protecteur du groupe carboxyle,
qui comprend la mise en réaction d'un composé de formule 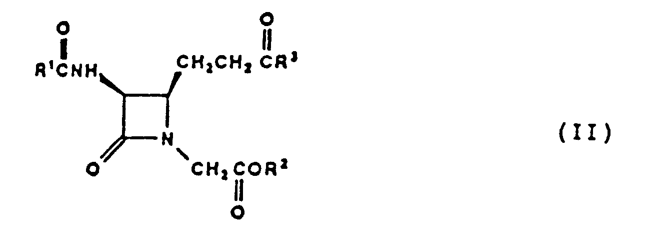 dans laquelle R¹ et R² sont tels que définis ci-dessus et R³ est un groupe qui se sépare, comprenant un substituant qui est déplacé de la molécule au cours de la réaction chimique et dont la base est un atome d'oxygène, avec une base forte non nucléophile comprenant le t-alcoxyde de lithium ou le t-alcoxyde de sodium.

2. Procédé selon la revendication 1, dans lequel R¹ représente un groupe benzyloxy.

3. Procédé selon la revendication 1 ou 2, dans lequel R² représente un groupe méthyle.

4. Procédé selon la revendication 1, 2 ou 3, dans lequel R² représente un groupe 4-nitrobenzyle.

5. Procédé selon l'une quelconque des revendications 1 à 4, dans lequel R³ représente un groupe phénoxy.

6. Produit intermédiaire de formule 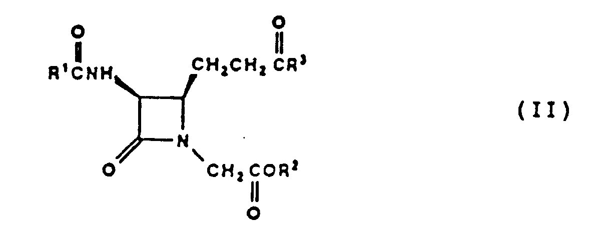 dans laquelle R¹ représente un groupe alkyle en C₁-C₆; un groupe phényle, 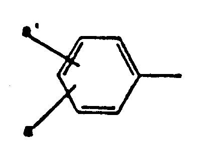 dans lequel a et a' représentent, indépendamment l'un de l'autre, un atome d'hydrogène, un groupe alkyle en C₁-C₄, un groupe alcoxy en C₁-C₄ ou encore un atome d'halogène; un groupe représenté par la formule 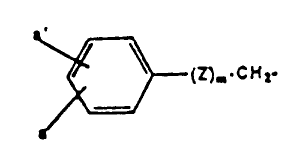 dans laquelle Z représente O ou S, m = 0 ou 1 et a et a' ont les mêmes significations que celles définies ci-dessus; ou bien R¹ représente R¹'O où R¹' représente un groupe alkyle en C₁-C₄, un groupe cycloalkyle en C₅-C₇, un groupe benzyle, un groupe nitrobenzyle, un groupe méthoxybenzyle ou un groupe halogénobenzyle;
R² représente un groupe protecteur du groupe carboxyle; et
R³ est un groupe qui se sépare.

7. Produit intermédiaire selon la revendication 6, dans lequel R¹ représente un groupe benzyloxy.

8. Produit intermédiaire selon la revendication 6, dans lequel R² représente un groupe 4-nitrobenzyle, un groupe méthyle ou un groupe 4-méthoxybenzyle.

9. Produit intermédiaire selon l'une quelconque des revendications 6 à 8, dans lequel R³ représente un groupe phénoxy, halogénophénoxy ou nitrophénoxy.

## Revendications (Revendications pour l'(les) Etat(s) contractant(s) suivant(s): ES)

1. Procédé pour préparer un composé de formule 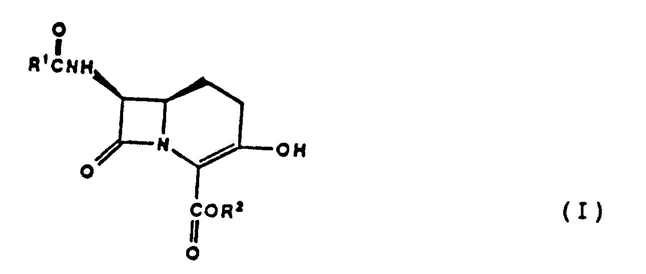 dans laquelle R¹ représente un groupe alkyle en C₁-C₆; un groupe phényle, 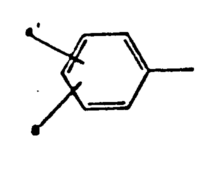 dans lequel a et a' représentent, indépendamment l'un de l'autre, un atome d'hydrogène, un groupe alkyle en C₁-C₄, un groupe alcoxy en C₁-C₄ ou encore un atome d'halogène; un groupe représenté par la formule 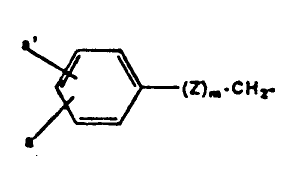 dans laquelle Z représente O ou S, m = 0 ou 1 et a et a' ont les mêmes significations que celles définies ci-dessus; ou bien R¹ représente R¹'O où R¹' représente un groupe alkyle en C₁-C₄, un groupe cycloalkyle en C₅-C₇, un groupe benzyle, un groupe nitrobenzyle, un groupe méthoxybenzyle ou un groupe halogénobenzyle; et R² représente un groupe protecteur du groupe carboxyle,
qui comprend la mise en réaction d'un composé de formule 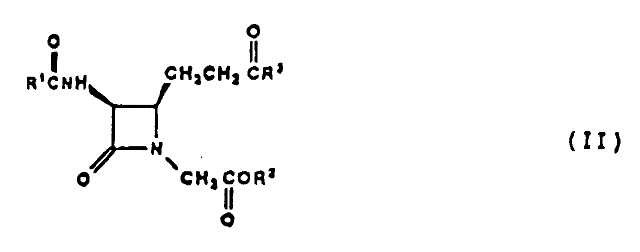 dans laquelle R¹ et R² sont tels que définis ci-dessus et R³ est un groupe qui se sépare, comprenant un substituant qui est déplacé de la molécule au cours de la réaction chimique et dont la base est un atome d'oxygène, avec une base forte non nucléophile Comprenant le t-alcoxyde de lithium ou le t-alcoxyde de sodium.

2. Procédé selon la revendication 1, dans lequel R¹ représente un groupe benzyloxy, un groupe phényle ou un groupe benzyle.

3. Procédé selon la revendication 1 ou 2, dans lequel R² représente un groupe méthyle.

4. Procédé selon la revendication 1, 2 ou 3, dans lequel R² représente un groupe 4-nitrobenzyle.

5. Procédé selon l'une quelconque des revendications 1 à 4, dans lequel R³ représente un groupe phénoxy, halogénophénoxy ou nitrophénoxy.
